# EUROPEAN PATENT SPECIFICATION

(11) **EP 4 100 521 B1**
(45) Date of publication and mention of the grant of the patent: **30.04.2025**
(21) Application number: 21751401.7
(22) Date of filing: 26.01.2021
(51) Int. Cl.: C12N 15/10, B01F 33/451, H01F 1/44, H01F 7/06, H01F 7/20, B03C 1/01, B03C 1/033, B03C 1/28

(54) **MAGNETIC MANIPULATION THROUGH SOLID-STATE METHOD AND APPARATUS**
MAGNETISCHE MANIPULATION DURCH FESTKÖRPERVERFAHREN UND VORRICHTUNG
MANIPULATION MAGNÉTIQUE AU MOYEN D'UN PROCÉDÉ ET D'UN APPAREIL À L'ÉTAT SOLIDE

(30) Priority: 04.02.2020 US 202062969713 P
(43) Date of publication of application: 14.12.2022
(73) Proprietor: Siemens Healthcare Diagnostics, Inc., Tarrytown, NY 10591 (US)
(72) Inventor: CHIA, Philip, El Cerrito, California 94530 (US); WOLDEMARIAM, Melaku, San Leandro, California 94578 (US); BLIZARD, Ben, Oakland, California 94610 (US)
(74) Representative: Schweitzer, Klaus
(86) International application number: PCT/US2021/070079
(87) International publication number: WO 2021/159135

(56) References cited:
- US-A- 5 546 006
- US-A- 5 546 006
- US-A1- 2014 135 198
- US-A1- 2014 135 198
- US-A1- 2015 300 938
- US-A1- 2015 300 938
- US-A1- 2018 369 831
- US-A1- 2020 011 773
- US-A1- 2020 011 773

## Description

### RELATED APPLICATIONS

This patent application claims priority from United States Provisional Patent Application No. 62/969,713, filed Feb. 4, 2020.

### FIELD OF THE DISCLOSURE

The disclosure herein relates generally to the field of cell lysing and nucleic acid purification and isolation. More particularly, the present disclosure relates to devices and methods for mechanically mixing a magnetic microparticles in a sample fluid.

### BACKGROUND

Cell lysis and nucleic acid isolation may use magnetic beads, or microparticles, to mix solutions and separate nucleic acids from solution. When isolating DNA, the dose per assay of microparticles used for nucleic acid transfer impacts assay performance. Variation in the microparticle dose can cause less or more nucleic acid to be bound, transferred, and then released (eluted) for use in the reaction. Accurate and repeatable control of the dosing is important to assay performance, in particular repeatability. This invention could provide more consistent dosing.

Typically, mechanical means of mixing ferrous microparticles (magnetic beads) with a suspending fluid requires moving parts. This undermines instrument reliability due to wear. It also increases operating noise, as well as the risk of splattering and aerosolization of biohazardous samples.

US Published Patent Applications 2013/0217144 and 2017/0284922 describe a method and apparatus for mixing magnetic particles in a microfluidic chamber on a chip using alternating 4-pole electromagnets. As a sample fluid enters the microfluidic chamber containing ferromagnetic and superparamagnetic particles from one edge, the particles rotate and mix in a two-dimensional plane as the fluid flows through to the outlet of the chamber. Magnets are electromagnetically actuated at various strengths and frequencies to manipulate both the ferromagnetic and superparamagnetic particles. This system requires a special purpose cartridge and only works with very small sample sizes.

It is known in the art methods and apparatus for processing fluids using electromagnetic structures configured to manipulate magnetic particles disposed within the fluids (US 2020/0011773 A1). The magnetic structures may be formed as a plurality of electromagnets configured to be individually actuated by a controller. Each of the electromagnets may generate a magnetic field within the fluid container. The electromagnets may be differentially actuated to create a magnetic field gradient within the fluid container to agitate, mix, or otherwise influence magnetic particles disposed within the fluid container.

### SUMMARY

According to the invention as specified in claim 1, a system for manipulating magnetic beads for processes involving molecular manipulations includes a housing for receiving a vessel containing a fluid sample and a plurality of magnetic beads therein, the housing having a circumference and a height perpendicular to the circumference; a first plurality of electromagnets spaced evenly around the circumference of the housing at a height *h₁*; a second plurality of electromagnets spaced evenly around the circumference of the housing at a height *h₂*, the second plurality of electromagnets being equal in number to the first plurality of electromagnets, the electromagnets of the second plurality offset from the electromagnets of the first plurality around the circumference of the housing; and a controller for selectively energizing electromagnets of the first and second pluralities in sequence causing the magnetic beads to circulate in the vessel.

The controller may include a microcontroller and an H-bridge that may independently energize or reverse the polarity the electromagnets, individually or in groups. Both the first and second pluralities of electromagnets may include three electromagnets, in embodiments.

The system includes an optical scattering sensor for measuring magnetic bead density in a target location within the vessel and providing the measured bead density to the controller to enable modification of the selective energizing of the electromagnets. In addition, the system may include a capacitance sensor for measuring magnetic bead density in a target location within the vessel and providing the measured bead density to the controller to enable modification of the selective energizing of the electromagnets.

According to the invention as specified in claim 8, a method of mixing magnetic beads in a fluid sample in a molecular analysis application may include depositing the fluid sample and magnetic beads within a vessel; positioning the vessel within a housing having a circumference and a height perpendicular to the circumference; disposing a first plurality of electromagnets evenly around the circumference of the housing at a height *h₁,* the electromagnets imparting a magnetic field within the vessel when energized; disposing a second plurality of electromagnet evenly around the circumference of the housing at a height *h₂,* the second plurality of electromagnets equal in number to the first plurality of electromagnets and each electromagnet of the second plurality offset from the electromagnets of the first plurality around the circumference of the housing, the electromagnets imparting a magnetic field within the vessel when energized; and selectively energizing the electromagnets of the first and second pluralities in a sequence causing the magnetic beads to circulate throughout the vessel.

The method may further include positioning electromagnets of the first plurality between electromagnets of the second plurality around the circumference of the housing.

The method may include selectively energizing by causing the magnetic beads to circulate around a vertical axis by energizing the first electromagnet; energizing the second electromagnet; energizing the third electromagnet; energizing the fourth electromagnet; energizing the fifth electromagnet; and energizing the sixth electromagnet.

In embodiments, the method may include selectively energizing by causing the magnetic beads to move up and down in the vessel by energizing the first electromagnet; energizing the fifth electromagnet; energizing the second electromagnet; energizing the sixth electromagnet; energizing the third electromagnet; and energizing the fourth electromagnet.

In embodiments, a method of drawing a sample having a quantity of magnetic beads from a sample fluid includes depositing the fluid sample and magnetic beads within a vessel; positioning the vessel within a housing having a circumference and a height perpendicular to the circumference, the housing comprising an array of electromagnets, the array comprising a first plurality of electromagnets spaced evenly around the circumference of the housing at a height *h₁,* and a second plurality of electromagnets spaced evenly around the circumference of the housing at a height *h₂,* the second plurality of electromagnets being equal in number to the first plurality of electromagnets, the electromagnets of the second plurality offset from the electromagnets of the first plurality around the circumference of the housing; selectively energizing the first and second pluralities of electromagnets in a sequence to cause the magnetic beads to circulate throughout the vessel; measuring magnetic bead density in a target location of the fluid within the vessel; modifying a sequence of energizing the first and second pluralities of electromagnets to maintain a consistent magnetic bead density in the target location; and drawing the sample from the target location of the fluid.

The method may include measuring magnetic bead density by measuring an optical scattering of the magnetic beads in the target location. In addition or alternatively, the method may include measuring a capacitance of the magnetic beads in the target location.

The sequence of selectively energizing the electromagnets may be chosen to cause the magnetic beads to rotate around the longitudinal axis of the vessel while simultaneously moving up and down within the vessel.

### BRIEF DESCRIPTION OF THE DRAWINGS

Illustrative embodiments of the disclosed technology are described in detail below with reference to the attached drawing figures:
FIG. 1 illustrates a perspective view of a solid state magnetic manipulation apparatus, in embodiments.
FIG. 2 is a side view of the apparatus of FIG. 1, in embodiments.
FIG. 3 is a top view of the apparatus of FIG. 1, in embodiments.
FIG. 4 is a bottom view of the apparatus of FIG. 1, in embodiments.
FIGS. 5A - 5F illustrate a process of mixing magnetic particles in the apparatus of FIG. 1, in embodiments.
FIG. 6 is a flowchart illustrating a method of solid-state magnetic manipulation, in embodiments.
FIG. 7 is a flowchart illustrating a method of measuring magnetic particle density, in embodiments.

### DETAILED DESCRIPTION

Disclosed herein is an apparatus and method for extracting nucleic acids such as DNA molecules from biological samples. In embodiments, no moving parts are used to vortex ferrous microparticles, which reduces the risk of splattering of the sample liquid and machine wear. It also reduces operating noise when compared to the traditional mechanical based mixing. In addition, this invention can utilize ferrous microparticles as a stirrer to mix heterogeneous liquids in low-cost and standard size tubes without the need of pipettes, microfluidic chips/channels, centrifuges, vortexers, or other mechanical devices and niche consumables. In embodiments, apparatus disclosed herein may be used as a measurement device to measure bead number density and modify magnetic patterns in order to deliver (closed-loop) consistent dosages in bead number. The terms magnetic beads, magnetic particles, nanoparticles and ferrofluid are used interchangeably herein.

In embodiments, magnetic beads may be magnetic micron- or nano-particles with a surface modification which binds target nucleic acids released during a sample preparation process of biological specimens. In the systems and methods described herein, a magnetic field is the only driving force for sample and/or magnetic bead handling.

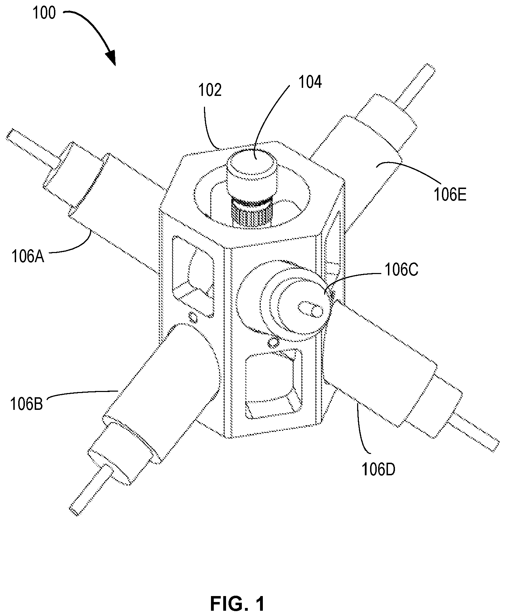

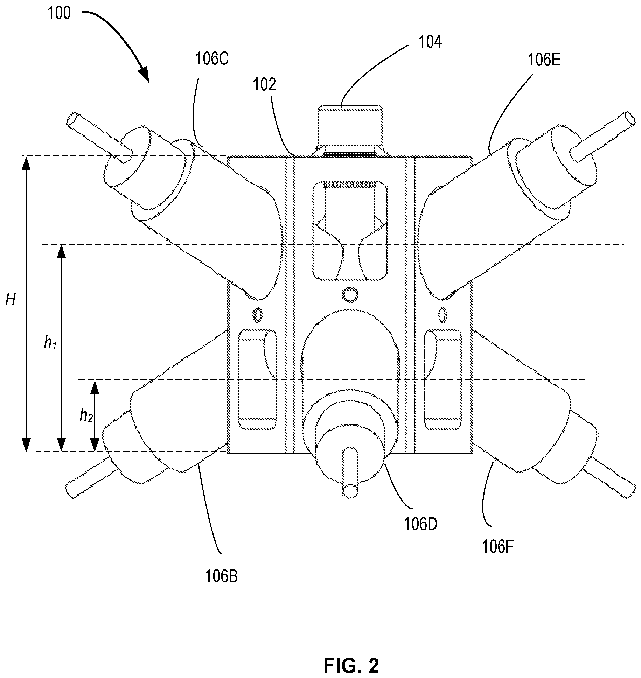

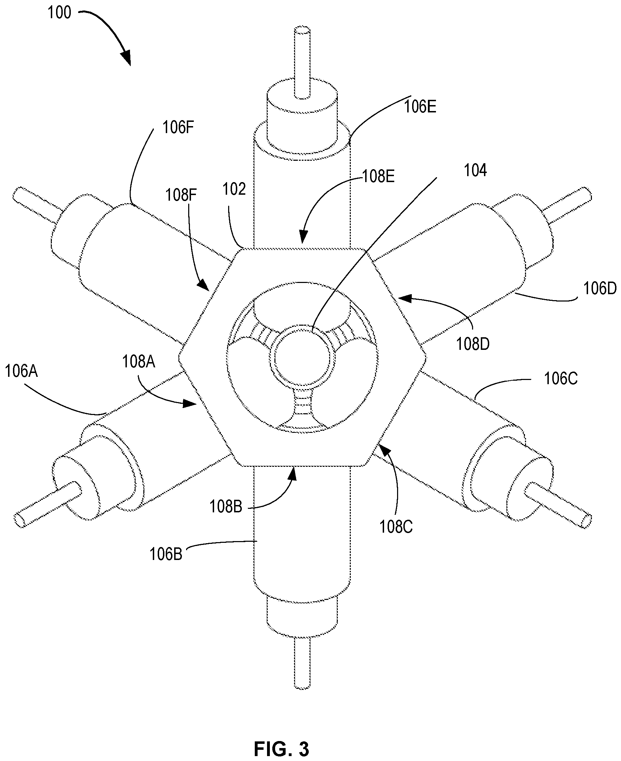

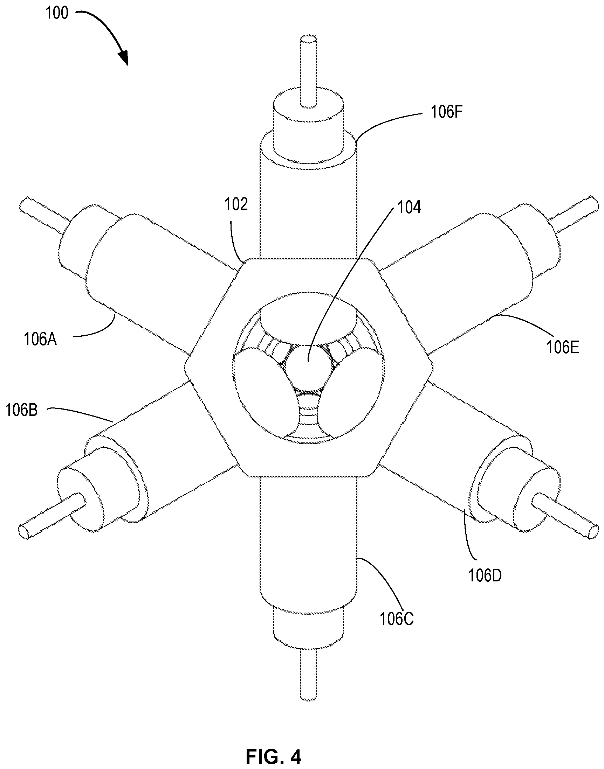

FIG. 1 illustrates a perspective view of an exemplary embodiment of an apparatus 100 for mixing fluids and magnetic particles in low cost and commercially available tube and vial consumables. FIG. 2 is a side view, FIG. 3 is a top view and FIG. 4 is a bottom view of the apparatus of FIG. 1, in embodiments. FIGS. 1 - 4 are best viewed together in the following description.

The apparatus 100 is comprised of a housing 102 for retaining a fluid sample tube 104. In embodiments, tube 104 may contain a 2mL fluid sample but apparatus 100 is not limited to any particular sample size. As shown in FIGS. 1 - 4, housing 102 is generally formed as a cylinder with six sides 108, and has a circumference and an overall height H. Each side 108 (FIG. 3) has an upper aperture in a horizontal plane at a height *h₁* and a lower aperture in a horizontal plane at a height *h₂*.

An array of six electromagnets is positioned radially around the circumference of housing 102. Although the electromagnets are shown with a central axis that is angled relative to the sides of the housing, this is not required. Three electromagnets 106A, 106C and 106E are arranged radially in the horizontal plane at a height *h₁* from the bottom of housing 102 as shown in FIG. 2. In the horizontal plane at height *h₁,* electromagnets 106A, 106C and 106E are inserted through apertures in sides 108A, 108C and 108E respectively, to a position close to tube 104. Apertures in sides 108B, 108D and 108F are open.

Three electromagnets 106B, 106D and 106F are arranged radially in the horizontal plane at a height *h₂* from the bottom of housing 102. In the horizontal plane at height *h₂,* electromagnets 106B, 106D and 106F are inserted into apertures in sides 108B, 108D and 108F, respectively, of housing 102. These are sides 108 that have open apertures in the horizontal plane at height *h₁*. Electromagnets 106 alternate around the circumference of housing 102 such that electromagnet 106B, for example, is positioned between electromagnets 106A and 106C.

Electromagnets 106 may be energized sequentially or in groups to induce a wide variety of movements to magnetic beads in tube 104. Magnetic beads may be caused to spin from the base of the tube 104 to the top of the tube in a helical (three-dimensional vortex) motion. Other patterns of movement are contemplated, such as moving up and down vertically along the longitudinal axis of the tube or around the circumference of tube 104 in a horizontal plane, for example.

Electromagnets 106 may be, for example, 12-24VDC electromagnets. A controller (not shown), may be used to switch electromagnets 106 ON and OFF. An "ON" state indicates that the electromagnet is generating a magnetic field and an "OFF" state means that it is not generating a magnetic field. In embodiments, electromagnets 106 may be controlled with any programmable control device, such as an Arduino Mega (2560) microcontroller and an H-Bridge (L298N) with flyback diodes. The pulsed signal (~ 5Hz) generated from the Arduino Mega microcontroller triggers the H-Bridge to turn on as well as reverse the polarity of an individual electromagnet 106.

Although housing 102 is shown and discussed with a specific arrangement of sides and apertures, this is for purposes of illustration only. Housing 102 may have more or fewer sides. Electromagnets may be arranged in more than two horizontal planes and the heights of the planes may be evenly spaced along overall height *H* or have different spacing. A horizontal plane may have any number of electromagnets. Further, sides 108 may not include an aperture where no electromagnet is inserted. Further, a cross-section of housing 102 may be circular instead of having distinct sides.

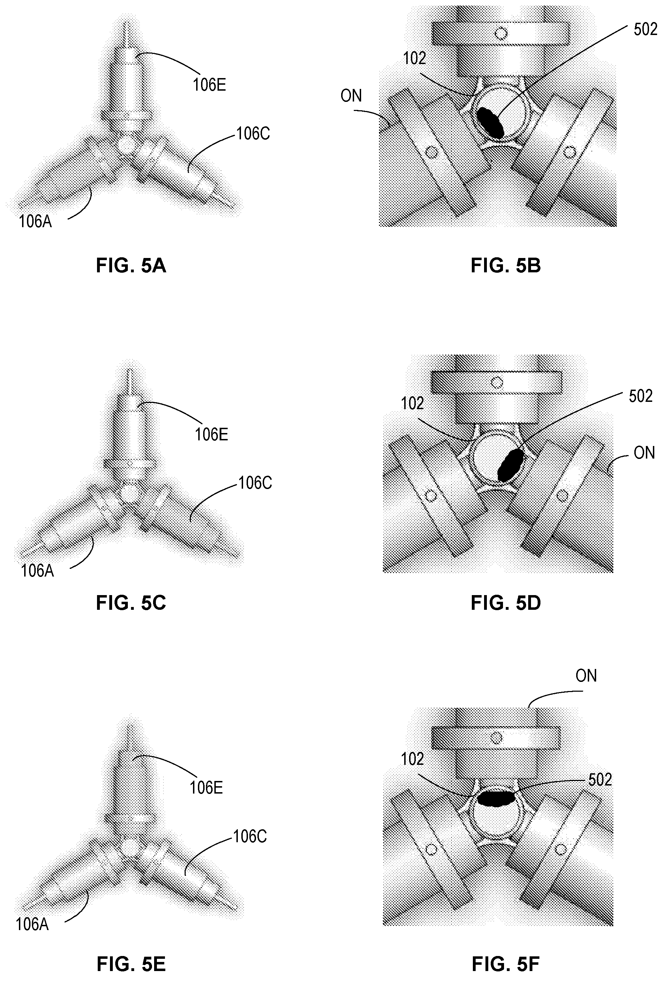

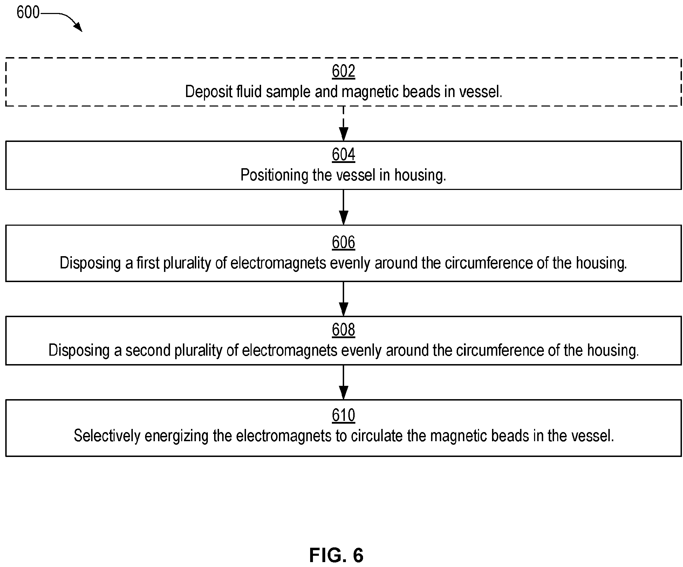

Operation of apparatus 100 to perform a solid-state mixing operation of magnetic particles will now be described. FIGS. 5A - 5F illustrate a movement of magnetic particles while FIG. 6 is a flowchart illustrating the method, in embodiments. FIGS. 5A-5F and 6 are best viewed together in the following discussion.

FIG. 6 is a flowchart of a method 600 of solid-state magnetic manipulation using apparatus 100 of FIG. 1. Not all steps need be practiced in the order described below, nor be utilized at all, depending upon the embodiment.

Step 602 includes depositing a fluid sample and magnetic beads in a vessel. In an example of step 602, a fluid sample may be any fluid containing a nucleic acid for analysis. Magnetic beads may be microparticles or nanoparticles, any ferromagnetic particle or superparamagnetic particle. The vessel may be a low cost and commercially available tube or vial consumables such as a 2mL tube. In embodiments, method 600 includes step 602 however, method 600 is not limited to placing the fluid sample and magnetic beads in a separate vessel.

Step 604 includes positioning a vessel in a housing. In an example of step 602, a vessel 104 containing a fluid sample and magnetic beads is placed in a housing 102.

Step 606 includes disposing a first plurality of electromagnets evenly around the circumference of the housing 102 at height *h₁.* In an example of step 606, housing 102 has six sides. Three sides have electromagnets disposed thereon at height *h₁* while the other three sides have no electromagnets at height *h₁,* as shown in FIGS. 1 - 4.

Step 608 includes disposing a second plurality of electromagnets evenly around the circumference of the housing 102 at height *h*₂. In an example of step 608, housing 102 has six sides. The three sides with no electromagnets in step 606 at height *h₁* have electromagnets 106B, 106D and 106F disposed thereon at height *h₂* while the other three sides have no electromagnets at height *h₂,* as shown in FIGS. 1 - 4.

Although two pluralities of three electromagnets are discussed herein, any number of electromagnets may be used as long as they are evenly spaced around a circumference of housing 102 in an arrangement that alternates electromagnets at height *h₁* and *h₂*.

Step 610 includes selectively energizing the electromagnets of the first and second pluralities in a sequence causing the magnetic beads to circulate throughout the vessel. In an example of step 610, the electromagnetic configuration described herein allows the beads to gradually spiral from the base of the tube to the top of the tube and back down. As shown in FIGS. 5A - 5F, one electromagnet is energized at a time to move magnetic beads through the interior of vessel 104. As shown in FIG. 5A, electromagnet 106A on the bottom left is turned ON. FIG. 5B shows a close-up view of the center of FIG. 5A where magnetic beads 502 have been drawn to electromagnet 106A. Although FIGS. 5A- 5F are discussed with reference to electromagnets 106A, 106C and 106E, the discussion herein also applies to electromagnets 106B, 106D and 106F.

Continuing with step 610, in FIGS. 5C and 5D, electromagnet 106A is de-energized while electromagnet 106C is energized so that magnetic beads 502 are drawn to electromagnet 106C. In FIGS. 5E and 5F, electromagnet 106C is de-energized while electromagnet 106E is energized so that magnetic beads 502 are drawn to electromagnet 106E. Electromagnets around the circumference of housing 102 are energized in sequence (akin to the operating principle of a stepper motor) thus enabling the magnetic beads to circulate in vessel 104 one step at a time. The speed of magnetic bead circulation is dependent on the rate that the electromagnets sequentially turn on and off.

Embodiments described herein use no moving parts to vortex ferrous microparticles which reduces the risk of splattering of the sample liquid, reduces wear as well as reduces operating noise when compared to the traditional mechanical based mixing. In addition, apparatus 100 may utilize ferrous microparticles as a stirrer to mix heterogeneous liquids in standard size tubes without the need of pipettes, microfluidic chips/channels, centrifuges, vortexers, or other mechanical devices and niche consumables.

Further, apparatus 100 is completely solid-state, requires little to no maintenance, and uses no tubing, valves, pumps, and other fluidic devices that will wear and/or clog from use. Apparatus 100 may also be used as a miniaturized stir plate with the magnetic particles placed in the fluid in lieu of a conventional stir bar.

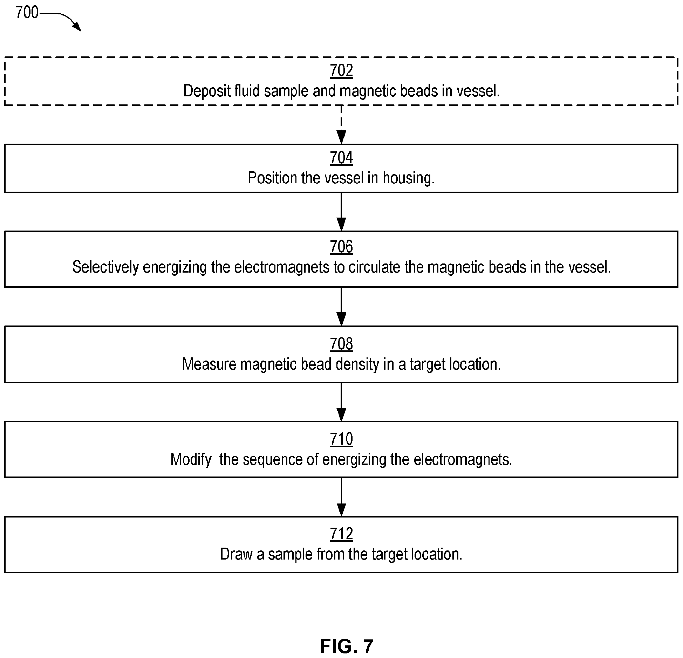

FIG. 7 is a flowchart illustrating a method 700 of measuring magnetic particle density using apparatus 100, in embodiments. Not all steps need be practiced in the order described below, nor be utilized at all, depending upon the embodiment. In addition to the apparatus discussed in connection with FIGS. 1 - 4, apparatus 100 used for the method of FIG. 7 would also include a physical measurement device such as camera having a light source and aperture.

Step 702 includes depositing a fluid sample and magnetic beads in a vessel. In an example of step 702, a fluid sample may be any fluid containing a nucleic acid for analysis. Magnetic beads may be microparticles or nanoparticles, any ferromagnetic particle or superparamagnetic particle. The vessel may be a low cost and commercially available tube or vial consumables such as a 2mL tube. In embodiments, method 700 includes step 702 however, method 700 is not limited to placing the fluid sample and magnetic beads in a separate vessel.

Step 704 includes positioning a vessel in a housing. In an example of step 702, a vessel 104 containing a fluid sample and magnetic beads is placed in a housing 102.

Step 706 includes disposing a first plurality of electromagnets evenly around the circumference of the housing 102 at height *h₁.* In an example of step 706, housing 102 has six sides. Three sides have electromagnets disposed thereon at height *h₁* while the other three sides have no electromagnets at height *h₁,* as shown in FIGS. 1 - 4.

Step 706 also includes disposing a second plurality of electromagnets evenly around the circumference of the housing 102 at height *h₂*. The three sides with no electromagnets in step 706 at height *h₁* have electromagnets 106B, 106D and 106F disposed thereon at height *h₂,* as shown in FIGS. 1 - 4.

Step 708 includes measuring magnetic bead density in a target location. In an example of step 708, a measuring device such as a camera, is positioned to measure magnetic bead density in vessel 104 while magnetic beads are being mixed in the vessel. In embodiments, measurement of bead number density in a target area may include optical scattering or capacitance. This measurement is fed back to the electromagnet control system to do a closed loop control of magnetic bead density so that a uniform bead dose may be sampled.

Step 710 includes modifying the sequence of energizing the electromagnets. In an example of step 710, this modification may maintain a uniform distribution of magnetic beads in the target location over time based on feedback from step 708. This modification may include changing the speed of energizing electromagnets, or the order in which they are energized. The sequence may mix the magnetic beads horizontally and/or vertically vessel 104, or switch between the two.

Step 712 includes drawing a sample from the target location. In an example of step 712, the modified sequence of step 710 changes the concentration of magnetic beads in the target location so that a known quantity of nucleic acid may be removed from vessel 104.

Changes may be made to embodiments described herein. Implementations of apparatus 100 may include physical rocking, shaking and inversions. Ultrasonic vibrations may be used to mix beads or generate a standing wave in appropriate acoustic environment.

Embodiments disclosed herein may be used, for example, in molecular diagnostics in nucleic acid extraction for real time PCR as well as an alternative to traditional mixing methods (e.g. pipette mixing, stir plate, etc.).

## Claims

1. A system for manipulating magnetic beads (502) for processes involving molecular manipulations, comprising:
a housing (102) receiving a vessel (104) containing a fluid sample and a plurality of magnetic beads (502) therein, the housing (102) having a circumference and a height perpendicular to the circumference;
a first plurality of electromagnets (106A-106F) spaced evenly around the circumference of the housing at a height *h₁;*
a second plurality of electromagnets spaced evenly around the circumference of the housing (102) at a height *h₂,* the second plurality of electromagnets being equal in number to the first plurality of electromagnets, the electromagnets of the second plurality offset from the electromagnets of the first plurality around the circumference of the housing (102);
a controller for selectively energizing electromagnets of the first and second pluralities in sequence causing the magnetic beads to circulate in the vessel (104); **characterised in that** the system comprises
an optical scattering sensor for measuring magnetic bead density in a target location within the vessel (104) and providing the measured bead density to the controller to enable modification of the sequence of selective energizing of the electromagnets (106A-106F).

2. The system of claim 1, wherein the controller further comprises a microcontroller and an H-bridge.

3. The system of claim 2, wherein the microcontroller generates a signal for independently turning the electromagnets (106A-106F) on and off individually or in groups.

4. The system of claim 2, wherein the microcontroller reverses the polarity of the electromagnets (106A-106F).

5. The system of claim 1, wherein the first plurality further comprises three electromagnets and the second plurality further comprises three electromagnets.

6. The system of claim 1, wherein the housing (102) holds the electromagnets (106A-106F) in position around the circumference of the vessel (104) when the vessel is received within the housing (102).

7. The system of claim 1, further comprising a capacitance sensor for measuring magnetic bead density in a target location within the vessel (104) and providing the measured bead density to the controller to enable modification of the selective energizing of the electromagnets (106A-106F).

8. A method of mixing magnetic beads (502) in a fluid sample in a molecular analysis application, comprising:
depositing the fluid sample and magnetic beads (502) within a vessel (104), as claimed in claim 1;
positioning the vessel (104) within a housing (102) having a circumference and a height perpendicular to the circumference;
disposing a first plurality of electromagnets evenly around the circumference of the housing at a height *h₁,* the electromagnets imparting a magnetic field within the vessel (104) when energized;
disposing a second plurality of electromagnet evenly around the circumference of the housing (102) at a height *h₂,* the second plurality of electromagnets equal in number to the first plurality of electromagnets and each electromagnet of the second plurality offset from the electromagnets of the first plurality around the circumference of the housing, the electromagnets imparting a magnetic field within the vessel when energized; and
selectively energizing the electromagnets (106A-106F) of the first and second pluralities in a sequence causing the magnetic beads to circulate throughout the vessel (104),
wherein the first plurality further comprises first, second and third electromagnets and the second plurality further comprises fourth, fifth and sixth electromagnets wherein the fourth electromagnet is positioned between the first and third electromagnets.

9. The method of claim 8, wherein selectively energizing further comprises causing the magnetic beads (502) to circulate around a vertical axis by:
energizing the first electromagnet;
energizing the second electromagnet;
energizing the third electromagnet;
energizing the fourth electromagnet;
energizing the fifth electromagnet; and
energizing the sixth electromagnet.

10. The method of claim 8, wherein selectively energizing further comprises causing the magnetic beads (502) to move up and down in the vessel (104) by:
energizing the first electromagnet;
energizing the fifth electromagnet;
energizing the second electromagnet;
energizing the sixth electromagnet;
energizing the third electromagnet; and
energizing the fourth electromagnet.

11. A method of drawing a sample having a quantity of magnetic beads (502), mixed according to claim 8, from a sample fluid, comprising:
depositing the fluid sample and magnetic beads (502) within a vessel (104);
positioning the vessel (104) within a housing (102) having a circumference and a height perpendicular to the circumference, the housing comprising an array of electromagnets (106A-106F), the array comprising a first plurality of electromagnets spaced evenly around the circumference of the housing (102) at a height *h₁,* and a second plurality of electromagnets spaced evenly around the circumference of the housing at a height *h₂,* the second plurality of electromagnets being equal in number to the first plurality of electromagnets, the electromagnets of the second plurality offset from the electromagnets of the first plurality around the circumference of the housing (102);
selectively energizing the first and second pluralities of electromagnets in a sequence to cause the magnetic beads (502) to circulate throughout the vessel (104);
measuring magnetic bead density in a target location of the fluid sample within the vessel (104);
modifying a sequence of energizing the first and second pluralities of electromagnets (106A-106F) to maintain a consistent magnetic bead density in the target location; and
drawing the sample from the target location of the fluid sample.

12. The method of claim 11, wherein measuring magnetic bead density further comprises measuring an optical scattering of the magnetic beads (502) in the target location.

13. The method of claim 11, wherein measuring magnetic bead density further comprises measuring a capacitance of the magnetic beads (502) in the target location.

14. The method of claim 11, wherein the sequence of selectively energizing the electromagnets (106A-106F) is chosen to cause the magnetic beads (502) to rotate around the longitudinal axis of the vessel (104) while simultaneously moving up and down within the vessel (104).

## Patentansprüche

1. System zum Manipulieren von Magnetkügelchen (502) für Prozesse, die molekulare Manipulationen beinhalten, umfassend:
ein Gehäuse (102), das ein Gefäß (104) aufnimmt, das eine Fluidprobe und eine Vielzahl von Magnetkügelchen (502) darin enthält, wobei das Gehäuse (102) einen Umfang und eine Höhe senkrecht zum Umfang aufweist;
eine erste Vielzahl von Elektromagneten (106A-106F), die gleichmäßig um den Umfang des Gehäuses in einer Höhe *h*₁ beabstandet sind;
eine zweite Vielzahl von Elektromagneten, die gleichmäßig um den Umfang des Gehäuses (102) in einer Höhe *h₂* beabstandet sind, wobei die zweite Vielzahl von Elektromagneten in der Anzahl gleich der ersten Vielzahl von Elektromagneten ist, wobei die Elektromagnete der zweiten Vielzahl von den Elektromagneten der ersten Vielzahl um den Umfang des Gehäuses (102) versetzt sind;
eine Steuerung zum selektiven Erregen von Elektromagneten der ersten und der zweiten Vielzahl nacheinander, wodurch bewirkt wird, dass die Magnetkügelchen in dem Gefäß (104) zirkulieren; **dadurch gekennzeichnet, dass** das System umfasst
einen optischen Streusensor zum Messen der Magnetkügelchendichte an einem Zielort innerhalb des Gefäßes (104) und Bereitstellen der gemessenen Kügelchendichte an die Steuerung, um eine Modifikation der Sequenz des selektiven Erregens der Elektromagnete (106A-106F) zu ermöglichen.

2. System nach Anspruch 1, wobei die Steuerung ferner einen Mikrocontroller und eine H-Brücke umfasst.

3. System nach Anspruch 2, wobei der Mikrocontroller ein Signal zum unabhängigen Ein- und Ausschalten der Elektromagnete (106A-106F) einzeln oder in Gruppen erzeugt.

4. System nach Anspruch 2, wobei der Mikrocontroller die Polarität der Elektromagnete (106A-106F) umkehrt.

5. System nach Anspruch 1, wobei die erste Vielzahl ferner drei Elektromagnete umfasst und die zweite Vielzahl ferner drei Elektromagnete umfasst.

6. System nach Anspruch 1, wobei das Gehäuse (102) die Elektromagnete (106A-106F) in einer Position um den Umfang des Gefäßes (104) hält, wenn das Gefäß innerhalb des Gehäuses (102) aufgenommen wird.

7. System nach Anspruch 1, ferner umfassend einen Kapazitätssensor zum Messen der Magnetkügelchendichte an einem Zielort innerhalb des Gefäßes (104) und Bereitstellen der gemessenen Kügelchendichte an die Steuerung, um eine Modifikation des selektiven Erregens der Elektromagnete (106A-106F) zu ermöglichen.

8. Verfahren zum Mischen von Magnetkügelchen (502) in einer Fluidprobe in einer Molekularanalyseanwendung, umfassend:
Abscheiden der Fluidprobe und der Magnetkügelchen (502) innerhalb eines Gefäßes (104) nach Anspruch 1;
Positionieren des Gefäßes (104) innerhalb eines Gehäuses (102), das einen Umfang und eine Höhe senkrecht zum Umfang aufweist;
Anordnen einer ersten Vielzahl von Elektromagneten gleichmäßig um den Umfang des Gehäuses in einer Höhe *h₁,* wobei die Elektromagnete ein Magnetfeld innerhalb des Gefäßes (104) erzeugen, wenn sie erregt werden;
Anordnen einer zweiten Vielzahl von Elektromagneten gleichmäßig um den Umfang des Gehäuses (102) in einer Höhe *h₂,* wobei die zweite Vielzahl von Elektromagneten in der Anzahl gleich der ersten Vielzahl von Elektromagneten ist und jeder Elektromagnet der zweiten Vielzahl von den Elektromagneten der ersten Vielzahl um den Umfang des Gehäuses versetzt ist, wobei die Elektromagnete ein Magnetfeld innerhalb des Gefäßes erzeugen, wenn sie erregt werden; und
selektives Erregen der Elektromagnete (106A-106F) der ersten und der zweiten Vielzahl in einer Sequenz, wodurch bewirkt wird, dass die Magnetkügelchen im gesamten Gefäß (104) zirkulieren,
wobei die erste Vielzahl ferner einen ersten, einen zweiten und einen dritten Elektromagnet umfasst und die zweite Vielzahl ferner einen vierten, einen fünften und einen sechsten Elektromagnet umfasst, wobei der vierte Elektromagnet zwischen dem ersten und dem dritten Elektromagnet positioniert ist.

9. Verfahren nach Anspruch 8, wobei das selektive Erregen ferner ein Bewirken umfasst, dass die Magnetkügelchen (502) um eine vertikale Achse zirkulieren, durch:
Erregen des ersten Elektromagnets;
Erregen des zweiten Elektromagnets;
Erregen des dritten Elektromagnets;
Erregen des vierten Elektromagnets;
Erregen des fünften Elektromagnets und
Erregen des sechsten Elektromagnets.

10. Verfahren nach Anspruch 8, wobei das selektive Erregen ferner ein Bewirken umfasst, dass sich die Magnetkügelchen (502) in dem Gefäß (104) nach oben und unten bewegen, durch: Erregen des ersten Elektromagnets;
Erregen des fünften Elektromagnets;
Erregen des zweiten Elektromagnets;
Erregen des sechsten Elektromagnets;
Erregen des dritten Elektromagnets und
Erregen des vierten Elektromagnets.

11. Verfahren zum Nehmen einer Probe, die eine Menge an Magnetkügelchen (502) aufweist, die nach Anspruch 8 gemischt sind, aus einem Probenfluid, umfassend:
Abscheiden der Fluidprobe und der Magnetkügelchen (502) innerhalb eines Gefäßes (104);
Positionieren des Gefäßes (104) innerhalb eines Gehäuses (102), das einen Umfang und eine Höhe senkrecht zum Umfang aufweist, wobei das Gehäuse eine Anordnung von Elektromagneten (106A-106F) umfasst, wobei die Anordnung eine erste Vielzahl von Elektromagneten, die gleichmäßig um den Umfang des Gehäuses (102) in einer Höhe h₁ beabstandet sind, und eine zweite Vielzahl von Elektromagneten, die gleichmäßig um den Umfang des Gehäuses in einer Höhe *h₂* beabstandet sind, umfasst, wobei die zweite Vielzahl von Elektromagneten in der Anzahl gleich der ersten Vielzahl von Elektromagneten ist, wobei die Elektromagnete der zweiten Vielzahl von den Elektromagneten der ersten Vielzahl um den Umfang des Gehäuses versetzt sind (102);
selektives Erregen der ersten und der zweiten Vielzahl von Elektromagneten in einer Sequenz, um zu bewirken, dass die Magnetkügelchen (502) im gesamten Gefäß (104) zirkulieren;
Messen der Magnetkügelchendichte an einem Zielort der Fluidprobe innerhalb des Gefäßes (104);
Modifizieren einer Sequenz des Erregens der ersten und der zweiten Vielzahl von Elektromagneten (106A-106F), um eine konsistente Magnetkügelchendichte am Zielort beizubehalten; und
Nehmen der Probe aus dem Zielort der Fluidprobe.

12. Verfahren nach Anspruch 11, wobei das Messen der Magnetkügelchendichte ferner ein Messen einer optischen Streuung der Magnetkügelchen (502) am Zielort umfasst.

13. Verfahren nach Anspruch 11, wobei das Messen der Magnetkügelchendichte ferner ein Messen einer Kapazität der Magnetkügelchen (502) am Zielort umfasst.

14. Verfahren nach Anspruch 11, wobei die Sequenz des selektiven Erregens der Elektromagnete (106A-106F) gewählt wird, um zu bewirken, dass sich die Magnetkügelchen (502) um die Längsachse des Gefäßes (104) drehen, während sie sich gleichzeitig innerhalb des Gefäßes (104) nach oben und unten bewegen.

## Revendications

1. Système de manipulation de billes magnétiques (502) pour des processus impliquant des manipulations moléculaires, comprenant :
un boîtier (102) recevant un récipient (104) contenant un échantillon de fluide et une pluralité de billes magnétiques (502) dans celui-ci, le boîtier (102) ayant une circonférence et une hauteur perpendiculaire à la circonférence ;
une première pluralité d'électro-aimants (106A-106F) espacés régulièrement autour de la circonférence du boîtier à une hauteur *h_{1;}*
une deuxième pluralité d'électro-aimants espacés régulièrement autour de la circonférence du boîtier (102) à une hauteur *h₂,* la deuxième pluralité d'électro-aimants étant égale en nombre à la première pluralité d'électro-aimants, les électro-aimants de la deuxième pluralité étant décalés par rapport aux électro-aimants de la première pluralité autour de la circonférence du boîtier (102) ;
un contrôleur pour exciter sélectivement les électro-aimants des première et deuxième pluralités dans l'ordre, provoquant la circulation des billes magnétiques dans le récipient (104) ; **caractérisé en ce que** le système comprend
un capteur de diffusion optique pour mesurer une densité de billes magnétiques dans un emplacement cible à l'intérieur du récipient (104) et fournir la densité de billes mesurée au contrôleur pour permettre la modification de la séquence d'excitation sélective des électro-aimants (106A-106F).

2. Système de la revendication 1, dans lequel le contrôleur comprend en outre un microcontrôleur et un pont en H.

3. Système de la revendication 2, dans lequel le microcontrôleur génère un signal pour activer et désactiver indépendamment les électro-aimants (106A-106F) individuellement ou en groupes.

4. Système de la revendication 2, dans lequel le microcontrôleur inverse la polarité des électro-aimants (106A-106F).

5. Système de la revendication 1, dans lequel la première pluralité comprend en outre trois électro-aimants et la deuxième pluralité comprend en outre trois électro-aimants.

6. Système de la revendication 1, dans lequel le boîtier (102) maintient les électro-aimants (106A-106F) en position autour de la circonférence du récipient (104) lorsque le récipient est reçu dans le boîtier (102).

7. Système de la revendication 1 comprend en outre un capteur de capacité pour mesurer la densité de billes magnétiques dans un emplacement cible à l'intérieur du récipient (104) et fournir la densité mesurée des billes au contrôleur pour permettre la modification de l'excitation sélective des électro-aimants (106A-106F).

8. Procédé de mélange de billes magnétiques (502) dans un échantillon de fluide dans une application d'analyse moléculaire, comprenant les étapes consistant à :
déposer l'échantillon de fluide et les billes magnétiques (502) dans un récipient (104), comme indiqué dans la revendication 1 ;
positionner le récipient (104) à l'intérieur d'un boîtier (102) ayant une circonférence et une hauteur perpendiculaire à la circonférence ;
disposer une première pluralité d'électro-aimants uniformément autour de la circonférence du boîtier à une hauteur *h₁*, les électro-aimants transmettant un champ magnétique à l'intérieur du récipient (104) lorsqu'ils sont excités ;
disposer une deuxième pluralité d'électro-aimants de façon régulière autour de la circonférence du boîtier (102) à une hauteur *h₂,* la deuxième pluralité d'électro-aimants étant égale en nombre à la première pluralité d'électro-aimants et chaque électro-aimant de la deuxième pluralité étant décalé par rapport aux électro-aimants de la première pluralité autour de la circonférence du boîtier, les électro-aimants transmettant un champ magnétique à l'intérieur du récipient lorsqu'ils sont alimentés ; et
exciter sélectivement les électro-aimants (106A-106F) des première et deuxième pluralités dans une séquence faisant circuler les billes magnétiques dans le récipient (104),
dans lequel la première pluralité comprend en outre les premier, deuxième et troisième électro-aimants et la deuxième pluralité comprend en outre les quatrième, cinquième et sixième électro-aimants, et dans lequel le quatrième électro-aimant est placé entre le premier et le troisième électro-aimant.

9. Procédé de la revendication 8, dans lequel l'excitation sélective consiste en outre à faire circuler les billes magnétiques (502) autour d'un axe vertical par les étapes suivantes :
exciter le premier électro-aimant ;
exciter le deuxième électro-aimant ;
exciter le troisième électro-aimant ;
exciter le quatrième électro-aimant ;
exciter le cinquième électro-aimant ; et
exciter le sixième électro-aimant.

10. Procédé de la revendication 8, dans lequel l'excitation sélective consiste en outre à faire déplacer de haut en bas les billes magnétiques (502) dans le récipient (104) par les étapes suivantes :
exciter le premier électro-aimant ;
exciter le cinquième électro-aimant ;
exciter le deuxième électro-aimant ;
exciter le sixième électro-aimant ;
exciter le troisième électro-aimant ; et
exciter le quatrième électro-aimant.

11. Procédé de prélèvement d'un échantillon contenant une quantité de billes magnétiques (502), mélangées selon la revendication 8, à partir d'un fluide d'échantillon, comprenant les étapes consistant à :
déposer l'échantillon de fluide et les billes magnétiques (502) dans un récipient (104) ;
positionner le récipient (104) à l'intérieur d'un boîtier (102) ayant une circonférence et une hauteur perpendiculaire à la circonférence, le boîtier comprenant un réseau d'électro-aimants (106A-106F), le réseau comprenant une première pluralité d'électro-aimants espacés régulièrement autour de la circonférence du boîtier (102) à une hauteur *h₁,* et une deuxième pluralité d'électro-aimants espacés régulièrement autour de la circonférence du boîtier à une hauteur *h₂,* la deuxième pluralité d'électro-aimants étant égale en nombre à la première pluralité d'électro-aimants,
les électro-aimants de la deuxième pluralité sont décalés par rapport aux électro-aimants de la première pluralité sur la circonférence du boîtier (102) ;
exciter sélectivement les première et deuxième pluralités d'électro-aimants dans une séquence pour faire circuler les billes magnétiques (502) dans le récipient (104) ;
mesurer la densité de billes magnétiques dans un emplacement cible de l'échantillon de fluide à l'intérieur du récipient (104) ;
modifier la séquence d'excitation des premier et deuxième pluralités d'électro-aimants (106A-106F) afin de maintenir une densité de billes magnétiques constante à l'emplacement cible ; et
prélever l'échantillon à partir de l'emplacement cible de l'échantillon de fluide.

12. Procédé de la revendication 11, dans lequel la mesure de la densité de billes magnétiques comprend en outre la mesure de la diffusion optique des billes magnétiques (502) dans l'emplacement cible.

13. Procédé de la revendication 11, dans lequel la mesure de la densité de billes magnétiques comprend en outre la mesure de la capacité des billes magnétiques (502) dans l'emplacement cible.

14. Procédé de la revendication 11, dans lequel la séquence d'excitation sélective des électro-aimants (106A-106F) est choisie pour faire tourner les billes magnétiques (502) autour de l'axe longitudinal du récipient (104) tout en se déplaçant simultanément de haut en bas à l'intérieur du récipient (104).
